# EUROPEAN PATENT SPECIFICATION

(11) **EP 3 846 868 B1**
(45) Date of publication and mention of the grant of the patent: **14.08.2024**
(21) Application number: 18793264.5
(22) Date of filing: 06.09.2018
(51) Int. Cl.: A61L 31/06, A61L 31/14, A61L 31/16

(54) **MEMBRANE TO RE-ESTABLISH CONTINUITY OF INJURED BIOLOGICAL TISSUES**
MEMBRAN ZUR WIEDERHERSTELLUNG DER KONTINUITÄT VON VERLETZTEM BIOLOGISCHEM GEWEBE
MEMBRANE POUR RÉTABLIR LA CONTINUITÉ DE TISSUS BIOLOGIQUES ENDOMMAGÉS

(43) Date of publication of application: 14.07.2021
(73) Proprietor: BIO ON S.p.A., 10128 Torino (IT)
(72) Inventor: GANDINI, Marco, 10095 Grugliasco (TO) (IT); GIUSTO, Gessica, 10095 Grugliasco (TO) (IT); SAETTONE, Paolo, 40016 San Giorgio Di Piano (BO) (IT); CIFELLI, Mario, 40016 San Giorgio Di Piano (BO) (IT); COMES FRANCHINI, Mauro, 40016 San Giorgio Di Piano (BO) (IT)
(74) Representative: Bottero, Carlo
(86) International application number: PCT/IB2018/056805
(87) International publication number: WO 2020/049339

(56) References cited:
- WO-A1-00/56376
- WO-A1-2005/002472
- WO-A1-2017/153619
- US-A1- 2012 052 292
- US-A1- 2014 277 572

## Description

The present invention relates to a membrane for re-establishing the continuity of injured biological tissues. In particular, the present invention relates to a membrane for re-establishing the continuity of injured biological tissues, wherein said membrane comprises a non-woven fabric comprising fibers of a polyhydroxyalkanoate (PHA).

It is well known that in abdominal (veterinary and human) surgery there are basically two similar conditions, although very distant from each other in terms of anatomy and surgical technique, in which surgeons face a series of complications and factors that prevent a successful outcome of the surgical intervention.

These two conditions are represented by the anastomoses of the gastrointestinal tract and the incisional hernias. In fact, in both cases, the surgeon tries to re-establish the continuity of injured tissues by affixing foreign material to the organism represented by sutures or prosthetic meshes. This material favors the continuity of the tissues and therefore the healing. Healing is represented in both cases by the resistance of the final construct (tissue + surgical material) to tensile forces and is obtained by the apposition of collagen by fibroblasts. In both cases, in fact, the final success is determined by the correct apposition of collagen by fibroblasts and by its transformation into fibrous tissue.

### INTESTINAL ANASTOMOSIS

With regard to intestinal anastomosis, intestine healing comprises a series of delicate passages in which the mucosal barrier is re-established, and the tissue subjected to surgical insult heals. The healing phases follow four steps: inflammatory phase, lag phase, proliferation phase and remodeling phase, during which different physiological phenomena involve molecular and cellular mechanisms.

Modern intestinal anastomosis techniques are aimed at reducing interference with these phases and promoting their progression. The dehiscence of anastomosis is a multifactorial mechanism and cannot be attributed to a single cause or a predisposing factor and the search for a method to prevent this catastrophic event remains a current problem. Several solutions have been evaluated for preventive purposes. Furthermore, a variety of surgical supports were applied with different success: intraluminal stents to support the anastomotic site and extraluminal wraps to protect it externally.

The primary integrity of the intestinal anastomosis depends on the surgical technique, on the ability of the tissues to support the suture or the staples, and on the healing process. Many methods have been studied to increase the initial strength of the anastomosis, especially in the first post-operative days. The most commonly used method is the buttressing or reinforcement of the suture line with interposition of different materials (bovine pericardium, synthetic materials) between the intestine stumps. The application of intraluminal stents or the bandage of the anastomosis (wrap) with biomaterials was also tested. These methods favor the deposition of fibrin on the serosa. However, they have the drawback that, in some cases, the excess of healing turns into stenosis.

More specifically:
- buttressing: there are three main categories of buttressing, permanent buttressing (such as fibrin glue and polyglycolic acid), semi-permanent buttressing (such as bovine pericardium patches or small intestine patches) and non-resorbable buttressing (such as ePTFE);
- stenting: stents are used as a support to withstand internal pressure and longitudinal force, thus improving the strength of the anastomosis in the short term. The anastomosis is free of compressive forces and damages to submucosal vascularization and mesenteric vessels are minimized. These stents must be reabsorbable and may deliver drugs, but on their own they do not improve the repair and strength of the anastomosis and therefore cannot prevent dehiscence;
- wrap: a biomaterial is wrapped around the anastomotic site to provide protection and support to fibroblasts.

### INCISIONAL HERNIA

The incisional wound is that wound, made during surgery, on the wall of the abdomen (laparotomy) during surgical interventions in the intestine, the abdominal viscera, and in the female reproductive system. If the healing of this wound becomes complicated (infection, tension, tissue rupture), it can give rise to what is called incisional (or ventral) hernia. Incisional hernias occur in 20% of patients undergoing laparotomy. From the patient's point of view, a ventral hernia can cause pain, alteration of normal functions, and can increase in volume resulting in deformation of the abdomen and imprisonment or strangulation of the intestine or other abdominal organs. The only known cure is to repair the ventral hernia by further surgery.

A truly effective and univocally accepted treatment to reduce the complications of incisional wounds has not yet been found.

The only method of proven efficacy (even if not able to totally eliminate the problem) is the use of a perfect aseptic technique, possibly associated with suture threads that release antibiotics.

Incisional hernias derive from the lack of healing of the initially separated and then sutured tissues after laparotomy interventions. Following laparotomy, the surgical incision is closed by one or more suture planes, to which the construct resistance is initially and entirely entrusted. Following the initial inflammatory phase, fibroblasts begin to affix collagen which, giving rise then to fibrous tissue, shift the task of providing resistance to the construct from the suture thread to the tissues.

If during this phase adverse factors occur (infections, excessive tension, metabolic disorders), the construct may be broken down, causing the two margins of the initial incision to separate. This physical distance between the two margins causes the fibroblasts to no longer apply collagen effectively and the result is the formation of a hernia.

To remedy this possibility, the most effective method is the implantation of prosthetic material, typically in the form of membranes or meshes. These meshes act as a support and as a "bridge" for fibroblasts, which are able to re-establish the continuity of the collagen matrix from one side of the incision to the other and thus to remedy the initial lack of healing producing a solid fibrous tissue wall that incorporates the implanted material.

There are membranes or meshes of different materials, for example polypropylene, polyglycolic acid and dual-component membranes (polypropylene and Teflon). As can be seen, these materials are profoundly different from each other: from those that are slowly reabsorbable (polyglycolic acid) to non-resorbable (polypropylene) ones, from those that cause a strong inflammatory reaction and therefore a consistent affixing of fibrous tissue (polypropylene) with the risk of adhesions of intestinal tracts on the mesh itself, to those with a very reactive material (polypropylene) on one side combined with a non-adherent (Teflon) one on the other.

WO 2005/002472 A1 discloses a nerve guide comprising a tubular membrane or sheet containing oriented fibers of polyhydroxybutyrate (PHB), wherein the fibers have a diameter of 1000 nm to 20000 nm.

US 2014/277572 A1 discloses non-woven membranes for medical devices containing fibers of P4HB and copolymers thereof, produced by electrospinning and having a diameter of 10 nm to 10 µm.

WO 2017/153619 A1 discloses nerve guides containing oriented hybrid nanofibers comprising poly-3-hydroxybutyrate-co-3-hydroxyvalerate (PHBV) having a diameter of 300 to 1500 nm made by electrospinning.

US 2012/052292 A1 discloses medical devices, such as hernia meshes coated with a drug releasing polymer which includes ibuprofen, tetracycline, cefarzolin, paclitaxel and/or doxycycline.

WO 00/56376 A1 discloses P3HB-co-4HB polymers for use as mesh or patch, for example in the repair of abdominal wall defects or hernias.

This derives from the fact that, as often happens, there is not yet a completely effective method or material of choice to solve problems related to the repair of incisional hernias.

The Applicant has therefore posed the problem of developing a membrane to re-establish the continuity of injured biological tissues, using a material that has the capacity of promoting the healing of injured biological tissues relatively quickly, without inducing inflammatory or rejection reactions.

The Applicant has now found that this problem, and others which will be better illustrated hereafter, may be solved by using a membrane comprising a non-woven fabric comprising polyhydroxyalkanoate (PHA) fibers as hereinafter defined.

In a first aspect thereof, the present invention therefore relates to a membrane for re-establishing the continuity of injured biological tissues, as defined in appended claim 1. Preferably, said fibers have an average diameter comprised from 1000 nm to 3000 nm.

Therefore, in the context of the present description and of the accompanying claims, the terms "to re-establish the continuity of injured biological tissues" means that healing process, known to the skilled person in the art, mediated by the affixing of collagen by fibroblasts, in the proximity of the margins of the injury, whose purpose is to induce closure and therefore the healing of the injury itself.

In the context of the present description and of the accompanying claims, the term "non-woven fabric" refers to a textile structure consisting of fibers which are arranged in a substantially random manner, that is to say, without a predetermined order which is instead characteristic of the actual fabrics, which are obtained by weaving (crossing of weft and warp threads by loom) or knitwear.

Tests carried out by the Applicant (described in detail hereafter) have surprisingly shown that the membrane according to the present invention has the advantages of promoting the healing of injured biological tissues relatively quickly, without inducing excessive inflammatory or rejection reactions, thus demonstrating a high biocompatibility, thereby providing a valid support in the healing process of biological tissue injuries and preventing the formation of dehiscences and incisional hernias.

The membrane, in order to re-establish the continuity of an injured biological tissue, is preferably inserted into the injury and fixed, by means of one or more sutures, to the area adjacent to the tissue injury. Subsequently, the injury is closed by suture and the membrane, being near the injured area, promotes the healing process, thereby re-establishing the continuity of the injured biological tissue.

In the context of the present description and of the accompanying claims, the term "biological tissue" refers to a set of cells, structurally similar, associated by function. It constitutes a higher level of cellular organization, intended to play a decisive role in an animal organism. According to the present invention, preferred biological tissues are: epithelial tissue, muscle tissue, nerve tissue, connective tissue. Even more preferably, the biological tissue is epithelial tissue.

According to the purposes of the present invention, the PHA containing 3-hydroxybutyrate monomer units which forms the fibers of the non-woven fabric is a poly-3-hydroxybutyrate homopolymer (P3HB)

In general, PHAs suitable for the present invention are homopolymers consisting of 3-hydroxybutyrate monomer units:

-O-CH(CH₃)-CH₂-CO- (I)

Also suitable but not part of the invention as claimed are copolymers of 3-hydroxybutyrate monomer units with at least one hydroxyalkanoate monomer unit having the formula

-O-CHR₁-(CH₂)ₙ-CO- (II)

wherein:
R₁ is selected from: -H and C₁-C₁₂ alkyl;
n is zero or an integer comprised in the range from 1 to 6, and is preferably 1 or 2; with the proviso that, when R₁ is methyl, n is different from 1. Preferably, R₁ is methyl or ethyl. Preferably, n is 1 or 2.

These copolymers, which are not according to the invention as claimed, preferably contain at least 20 mol%, more preferably at least 30 mol%, of 3-hydroxybutyrate monomer units, the remainder being hydroxyalkanoate monomer units other than 3-hydroxybutyrate. In such copolymers, the quantity of 3-hydroxybutyrate monomer units is generally equal to or less than 99 mol%, preferably equal to or less than 90 mol%. Suitable hydroxyalkanoates monomer units other than 3-hydroxybutyrate are those deriving from: 4-hydroxybutyrate, 3-hydroxyvalerate, 3-hydroxyhexanoate, 3-hydroxyoctanoate, 3-hydroxyundec-10-enoate, 4-hydroxyvalerate. These are not part of the invention as claimed.

In the case of copolymers of PHA, which are not according to the invention as claimed, they are preferably selected from: poly(3-hydroxybutyrate-co-3-hydroxyvalerate) (P3HBV), poly(3-hydroxybutyrate-co-3-hydroxyhexanoate) (P3HBH), poly(3-hydroxybutyrate-co-4-hydroxybutyrate), poly(3-hydroxybutyrate-co-3-hydroxyvalerate-co-4-hydroxyvalerate (P3HBVV), or mixtures thereof.

According to the purposes of the present invention, the PHA is a poly-3-hydroxybutyrate homopolymer (P3HB).

PHAs suitable for the present invention preferably have a weight average molecular weight (Mw) comprised in the range from 5,000 to 1,500,000 Da, more preferably from 100,000 to 1,000,000 Da. The weight average molecular weight can be determined according to known techniques, in particular by means of GPC (Gel Permeation Chromatography) analysis.

The Applicant believes that the use of PHA fibers containing 3-hydroxybutyrate monomeric units, and in particular poly-3-hydroxybutyrate fibers (P3HB), ensures a substantially complete resorption of the fibers themselves by the organism in which the membrane has been implanted, without any toxic or harmful effect.

In fact, when the PHA containing 3-hydroxybutyrate monomer units is metabolized within the organism, the 3-hydroxybutyric acid monomer is released, which, due to the presence of an OH group on the carbon in beta position, undergoes a dehydration reaction catalysed by some enzymes, resulting in the formation of crotonic acid (an alpha-beta-unsaturated acid). Within the organism, the crotonic acid undergoes a reaction of addition of hydrogen with consequent formation of butyric acid (a "Short Chain Fatty Acid" SCFA that is easily assimilated by the organism). This metabolic pathway is impossible with other monomers derived from other PHAs, such as with 4-hydroxybutyric acid which is generated as a result of the metabolization of poly-4-hydroxybutyrate (P4HB): in fact, in 4-hydroxybutyric acid the OH group is positioned on the gamma carbon and not on the beta carbon, thus making it impossible the formation of crotonic acid by dehydration.

The PHA fibers of the non-woven fabric according to the present invention are produced by electrospinning. In the context of the present description and of the accompanying claims, "electrospinning" means a spinning method, known in the art, which exploits the interaction that is created between a polymeric solution and an external electric field. Electrospinning makes it possible to produce very small diameter filaments, generally of the order of tens or hundreds of microns, with high speed and accurate process control, a result difficult to obtain with common fiber production techniques by extrusion spinning.

An electrospinning system mainly comprises a pump connected to a syringe containing the polymer solution, a spinneret connected to the syringe, a high voltage source and a manifold. Due to the action of the pump, the polymer solution is pushed outside the syringe, towards the manifold, passing through the spinneret with a constant and controllable flow. When a high potential difference (usually between 1 and 30 kV) is applied between the spinneret and the manifold, a drop of polymer is generated at the tip of the spinneret. As the difference in potential increases, the drop is subjected to increasing repulsive forces between its surface charges and the electrostatic forces exerted by the external electric field, up to the distortion of the drop itself with the formation of a cone commonly known as Taylor's cone. As soon as the electric field exceeds a critical value, specific for each polymeric solution, the electrostatic forces prevail over the surface tension leading to the formation of a polymeric fiber or thread.

The non-woven fabric according to the invention is made by electrospinning, with dimensions suitable for surgical needs, that is to say, depending on the length and the extent of the injury to be treated. The non-woven fabric has dimensions comprised from 1 cm² to 50 cm², a rectangular, rhomboidal, square, oval, round or irregular shape, and a thickness comprised from 10 um to 700 um, preferably comprised from 50 um to 500 um.

According to another aspect thereof, the present invention relates to a method for producing a membrane comprising a non-woven fabric according to the present invention, which comprises:
- preparing a spinning solution by solubilization of the polyhydroxyalkanoate (PHA) containing 3-hydroxybutyrate monomeric units in an organic solvent;
- subjecting the spinning solution to an electrospinning process by means of a spinneret and a rotating support placed substantially perpendicular to the spinning direction, , using a configuration called "horizontal arrangement", so as to obtain the non-woven fabric.

The organic solvent that can be used for the preparation of the spinning solution may be selected from those in which the PHA is soluble and which also have features of adequate electrical conductivity, polarity, vapor pressure, etc., such as 1,1,1,3,3,3-hexafluoro-2-propanol (HFIP), chloroform, N,N-dimethylformamide (DMF), or mixtures thereof.

In the spinning solution, the PHA containing 3-hydroxybutyrate monomer units preferably has a concentration comprised from 1% to 20% w/v, more preferably comprised from 2% w/v to 10% w/v.

According to a preferred embodiment of the membrane according to the present invention, at least one active ingredient is associated with the fibers of the non-woven fabric of the membrane.

The at least one active ingredient which may be associated with the fibers of the non-woven fabric is preferably selected from: pentoxifylline, doxycycline, ibuprofen, cefazolin, rifampicin, paclitaxel, itraconazole, mefoxin, tetracycline hydrochloride or mixtures thereof.

Pentoxifylline is one of the most important xenobiotics, it has an effect on the perfusion of organs and tissues, on the deformability of erythrocytes and on the viscosity of the plasma. Many vasoactive substances are known to increase blood flow in tissues and, consequently, oxygenation. Pentoxifylline is one of these substances capable of increasing the flexibility, the ability to deform and the viscosity of the erythrocytes, decreasing the aggregation of the platelets.

Doxycycline is an antibiotic and a metalloprotease inhibitor. Several studies report an increase in matrix metalloproteases (MMP) activity in the anastomoses, with a consequent increase in the degradation of the local tissue present around the suture. Therefore, the administration of an active ingredient inhibiting of MMP leads to a decrease in the degradation of the tissue present around the suture.

Even cells and autologous products (Platelet Rich Plasma, PRP) can be associated with fibers of non-woven fabrics.

Preferably, the active ingredient, associated with fibers of the non-woven fabric, is doxycycline. Doxycycline is preferably present at a concentration comprised from 0.1% w/w to 4% w/w, more preferably comprised from 0.2% w/w to 2% w/w, with respect to the weight of the PHA.

In the case in which it is intended to produce a non-woven fabric comprising at least one active ingredient, preferably the spinning solution further comprises the at least one active ingredient. In particular, the at least one active ingredient may be associated with the fibers mainly in two ways:
- by inserting the at least one active ingredient into the spinning solution; in this case, the release time of the active ingredient from the non-woven fabric depends mainly on the compatibility between the active ingredient and the spinning solution;
- by inserting the at least one active ingredient into the electrospun PHA fibers; in this case, the active ingredient, suitably dissolved within the spinning solution, is co-axially electrospun, by means of a specific spinneret, together with the fibers; the active ingredient having insufficient time to recrystallize, remains trapped inside the fibers or in the form of an amorphous solid or solution.

The feature of providing a non-woven fabric comprising at least one active ingredient provides the advantage of giving the membrane according to the present invention a further therapeutic effect, specific according to the active ingredient used. For example, the use of doxycycline gives the therapeutic advantage of increasing the inhibition of MMPs. Said therapeutic effect is particularly advantageous in preventing the formation of dehiscences and incisional hernias, since, as described above, the administration of an active ingredient inhibiting of MMP, by inhibiting their action leads to a decrease in the degradation of the issue surrounding the suture. This decrease promotes the physiological healing process.

The following embodiment examples are provided purely for illustrative purposes of the present invention and should not be interpreted as limiting the scope of protection defined by the accompanying claims.

### EXAMPLE 1: Production of the P3HB non-woven fabric.

The purified P3HB was dissolved in chloroform so as to obtain a spinning solution with a concentration of 6% w/v. The solution was stirred on a heating plate at 800 rpm at a temperature of 80°C for about 2 hours in order to facilitate the complete dissolution of the polymer.

After complete dissolution, N,N-dimethylformamide (DMF) was added to the solution until a chloroform/DMF ratio of 90:10 v/v was achieved.

The solution was stirred at 800 rpm at a temperature of 25°C for about 2 hours.

Thereafter, the solution was cooled, poured into a 10 mL plastic syringe and placed inside the electrospinning system to be processed. The P3HB filament deposition was made by means of a spinneret and a rotating cylindrical support placed substantially perpendicular to the spinning direction, using a configuration called "horizontal arrangement". Once the deposition on the rotating support had been carried out, the deposition was opened and laid in order to obtain the non-woven fabric in the form of a flat membrane.

The deposition of the P3HB filament on the rotating cylindrical support therefore allowed obtaining the non-woven fabric.

The following parameters were used for the electrospinning process
Spinning solution: purified P3HB dissolved in chloroform/DMF (90:10 v/v) at 6% w/v;
solution volume processed: 120 mL;
solution rate: 8 mL/hour;
translational speed of the spinneret: 8 mm/s;
distance between the tip of the spinneret and the cylindrical support (gap): 15 cm;
voltage applied to the electrodes: 20 kV;
rotation speed of the cylindrical support: 150 rpm;
diameter of the spinneret outlet hole: 2.15 mm;

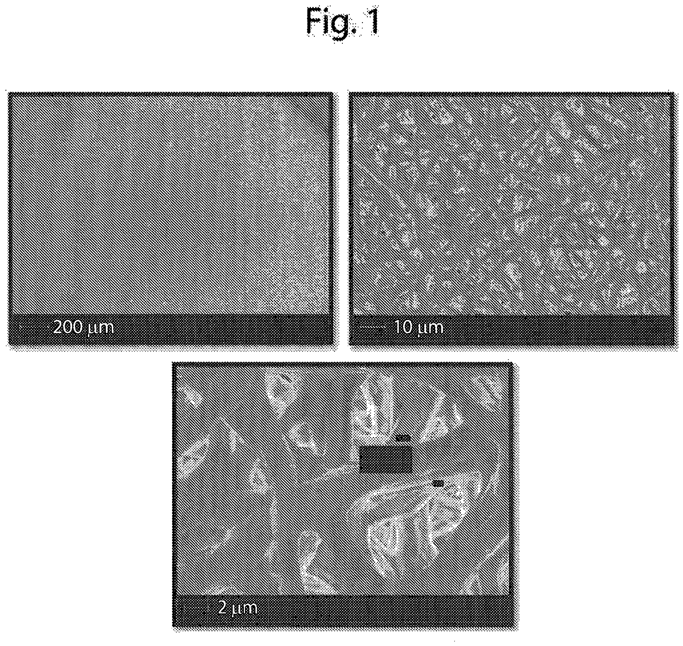

Figure 1 shows the images of the non-woven fabric of Example 1 obtained by scanning electronic microscopy (SEM)**.**

### EXAMPLE 2: Production of the P3HB and doxycycline non-woven fabric.

The purified P3HB was dissolved in chloroform so as to obtain a spinning solution with a concentration of 6% w/v. The solution was stirred on a heating plate at 800 rpm at a temperature of 80°C for about 2 hours in order to facilitate the complete dissolution of the polymer.

At the same time, a stock solution of doxycycline was prepared by dissolving the active ingredient in N,N-dimethylformamide (DMF). The resulting solution was stirred on a plate at 800 rpm at a temperature of 25°C for about 1 hours.

The stock solution of doxycycline in DMF was diluted three times in order to obtain three solutions having different concentrations of doxycycline:
- solution 1: P3HB (6% w/v) and doxycycline (0.1% w/v) in chloroform/DMF (90/10 v/v);
- solution 2: P3HB (6% w/v) and doxycycline (0.25% w/v) in chloroform/DMF (90/10 v/v);
- solution 3: P3HB (6% w/v) and doxycycline (0.5% w/v) in chloroform/DMF (90/10 v/v).

The three solutions were stirred at 800 rpm at a temperature of 25°C for about 2 hours.

Thereafter, the three solutions were made to cool, were respectively poured into three 10 mL plastic syringes and one at a time placed inside the electrospinning system to be processed in succession. The P3HB + doxycycline filament deposition was made by means of a spinneret and a rotating cylindrical support placed substantially perpendicular to the spinning direction, using a configuration called "horizontal arrangement". Once deposition on the rotating cylindrical support had been carried out, the deposition was opened and laid in order to obtain the non-woven fabric admixed with doxycycline in the form of a membrane. The deposition of the P3HB + doxycycline filament on the rotating cylindrical support therefore allowed obtaining the non-woven fabric added of the active ingredient.

The following parameters were used for the electrospinning process of solution 1:
spinning solution 1: purified P3HB (6% w/v) and doxycycline (0.1% w/v) dissolved in chloroform/DMF (90:10 v/v);
solution 1 volume processed: 50 mL;
solution 1 rate: 7 mL/hour;
translational speed of the spinneret: 6 mm/s;
distance between the tip of the spinneret and the cylindrical support (gap): 20 cm;
voltage applied to the electrodes: 20 kV;
movement speed of the flat support: 200 rpm;
diameter of the spinneret outlet hole: 2.15 mm;

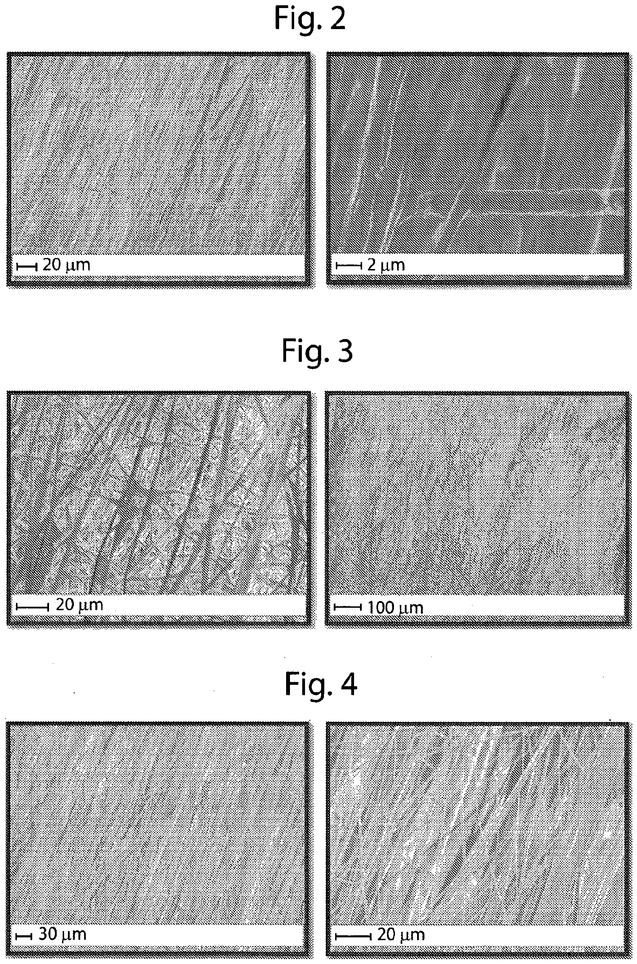

Figure 2 shows the images of the non-woven fabric added of doxycycline (0.1% w/v) obtained by scanning electronic microscopy (SEM).

The following parameters were used solution 2 for the electrospinning process of solution 2
spinning solution 2: purified P3HB (6% w/v) and doxycycline (0.25% w/v) dissolved in chloroform/DMF (90:10 v/v);
solution 2 volume processed: 50 mL;
solution 2 rate: 7 mL/hour;
translational speed of the spinneret: 6 mm/s;
distance between the tip of the spinneret and the cylindrical support (gap): 20 cm;
voltage applied to the electrodes: 20 kV;
rotation speed of the cylindrical support: 200 rpm;
diameter of the spinneret outlet hole: 2.15 mm;

Figure 3 shows the images of the non-woven fabric admixed with doxycycline (0.25% w/v) obtained by scanning electronic microscopy (SEM).

The following parameters were used solution 3 for the electrospinning process of solution 3
spinning solution 3: purified P3HB (6% w/v) and doxycycline (0.5% w/v) dissolved in chloroform/DMF (90:10 v/v);
solution 3 volume processed: 50 mL;
solution 3 rate: 7 mL/hour;
translational speed of the spinneret: 6 mm/s;
distance between the tip of the spinneret and the cylindrical support (gap): 20 cm;
voltage applied to the electrodes: 20 kV;
movement speed of the cylindrical support: 200 rpm;
diameter of the spinneret outlet hole: 2.15 mm;

Figure 4 shows the images of the non-woven fabric added of doxycycline (0.5% w/v) obtained by scanning electronic microscopy (SEM).

The concentration of doxycycline trapped within the three non-woven fabrics was then determined by the following method.

A rectangular portion of about 150 mg of weight was cut from each non-woven fabric. Each portion was in turn cut and reduced into small pieces, in order to promote the release of trapped doxycycline. The pieces of each non-woven fabric were immersed, within a 50 mL falcon, in 10 mL 0.1 M NaOH. The three suspensions obtained were stirred overnight on a rotating plate and subsequently put in centrifuge at 6000 rpm for 15 minutes. The supernatant solution of each falcon was diluted and analyzed by UV-Vis (results reported in Table 1).

**Table 1: concentration (% w/w) of doxycycline in the three non-woven fabrics**

| Sample | Theoretical doxycycline compared to the weight of P3HB (% w/w) | | Actual doxycycline compared to the weight of P3HB (% w/w) | | Efficiency (%) | |
|---|---|---|---|---|---|---|
| P3HBDOX1 | | 1.2% | | 0.5% | | 25% |
| P3HBDOX2 | | 4.2% | | 1% | | 21.5% |
| P3HBDOX3 | | 8.3% | | 3.6% | | 43.6% |

### EXAMPLE 3: Preclinical study - intraperitoneal implant of membranes comprising non-woven fabrics of Examples 1 and 2.

### a) Materials and methods

The study was conducted according to the good practices described in the following documents:
"ISO 10993-2:2006 Biological evaluation of medical devices Part 2: Animal welfare requirements";
"ISO 10993-6:2016 Biological evaluation of medical devices-Part 6: Tests for local effects after implantation";
"F763 - 04(2016) Standard Practice for Short-Term Screening of Implant Materials";
"F1904 - 14 Standard Practice for Testing the Biological Responses to Particles in vivo";
"F1983 - 14 Standard Practice for Assessment of Selected Tissue Effects of Absorbable Biomaterials for Implant Applications".

Adult Sprague-Dawley male rats weighing 225-250 g each were used.

42 animals (of the total 120 used) were randomly allocated to the groups:
- P3HB group (membrane comprising the non-woven fabric of Example 1);
- P3HB + Doxy group (membrane comprising the non-woven fabric of Example 2 in a 1:1000 ratio of doxycycline).

### b) Biocompatibility

The animals were subjected to an intraperitoneal implant of a portion of 1 cm² of membrane comprising non-woven fabric to be tested. After laparotomy on the midline, the membrane was implanted in the abdomen adhering to the parietal peritoneum about 1 cm to the left of the white line.

An incision of 2 cm was performed on the median plane comprising skin, subcutis and the white line.

The membrane was then implanted as described by "Azab AK, Doviner V, Orkin B, Kleinstern J, Srebnik M, Nissan A, Rubinstein A: Biocompatibility evaluation of crosslinked chitosan hydrogels after subcutaneous and intraperitoneal implantation in the rat; J Biomed Mater Res A 2007, Nov; 83(2):414-422". Briefly, a single 1 cm² portion was inserted through the surgical incision and fixated at about 1 cm from the white line to the left abdominal wall with a single simple suture point. The abdominal wound was then closed on two planes with a simple continuous suture with glycomer 631 USP 3-0 for the band and USP 3-0 nylon for the skin.

In the negative control group, the procedure was performed in the same way but without insertion of the membrane.

The surgical procedures lasted about 20 minutes each, while the anaesthesia time of each rat was about 1 hour.

During and after the procedures, the rats were kept on a heated plate and under an infrared lamp to avoid hypothermia and 5 ml of isotonic physiological solution (0.9%) were administered subcutaneously before putting them in their respective cages.

In the post-operative period, the administration of analgesics (Carprofen 5-10 mg/kg subcutaneously for 2-3 days), antibiotics (penicillin + dihydrostreptomycin 0.2-0.3 mg/kg subcutaneously every 2 days 2 times a day) was assured.

The subjects were kept in single cages and then subjected to euthanasia on day 14, and then for each rat the left abdominal wall, in the portion comprising the membrane, was removed and fixated in formalin. Thereafter, the tissue was dissected, stained with hematoxylin and eosin and examined by a pathologist not aware of the sample assignment group.

The following parameters were considered to histologically define the tissue response:
- neovascularization;
- fibrosis;
- adipose infiltration.

For each parameter, a score was assigned on a scale from 0 (absent) to 4 (very abundant).

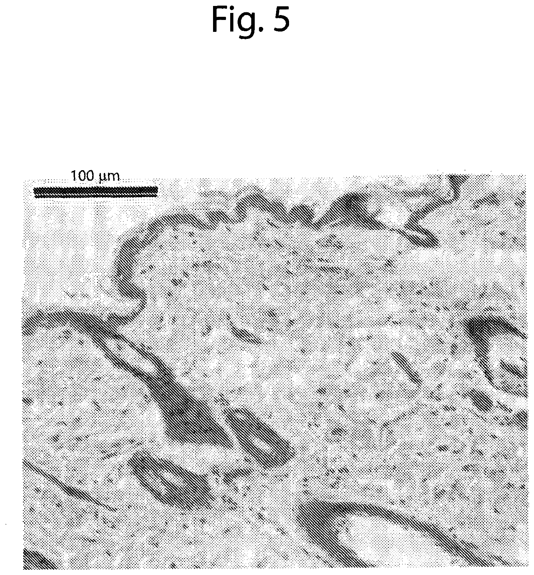

Figure 5 shows an example of a histological preparation of Example 3.

Furthermore, the expression of COX-2 (cyclooxygenase 2) was evaluated to define the degree of inflammation of the tissue.

All the data obtained were analyzed to verify the normality thereof by the Shapiro-Wilk test.

Non-parametric tests (Kruskal-Wallis) were then used to determine their significance.

### c) Results

Table 2 reports the results concerning neovascularization.

**Table 2 (neovascularization)**

| | P3HB | P3HB+Doxy | |
|---|---|---|---|
| Median (range) | 3(2-4)* | 2(1-2)* | **p*=0.0163 |
| IQ range | 2.25-3.08 | 1.67-2.12 | |

These results indicate that the two membranes comprising non-woven fabrics are comparable with respect to neovascularization.

Table 3 reports the results related to fibrosis.

**Table 3 (fibrosis)**

| | P3HB | P3HB+Doxy | |
|---|---|---|---|
| Median (range) | 3 (2-4) | 4(3-4)* | *p=0.0001 |
| IQ range | 2.8-3.53 | 3.49-4.1 | |

These results indicate that P3HB+DOXY induces a more pronounced fibrosis compared to P3HB. This is attributable to the fact that doxycycline has an inhibiting effect on the metalloproteases responsible for the inhibition of collagen remodeling.

Table 4 shows the results concerning adipose infiltration.

**Table 4 (adipose infiltration)**

| | P3HB | P3HB+Doxy | |
|---|---|---|---|
| Median (range) | 2.5(1-4)^{b} | 1(1-2)^{a,b} | ^{a}p=0.0143 |
| | | | ^{b}p=0.0156 |
| IQ range | 1.86-3.13 | 0.9-1.64 | |

These results indicate that P3HB+DOXY induces a lower adipose infiltration compared to P3HB. This result can also be explained by the inhibition of metalloproteases by doxycycline which, potentially, causes a substitution of adipose tissue with fibrous tissue.

COX-2 expression was not significantly different between the two groups (data not shown). This indicates that the degree of inflammation induced by the presence of the two membranes comprising the non-woven fabrics is comparable.

### d) Conclusions

The membrane comprising the P3HB+DOXY non-woven fabric was found to induce a superior tissue response compared to the membrane comprising the P3HB non-woven fabric, causing greater fibrous tissue apposition.

Not having shown any signs of toxicity and not having caused reactions different from those expected for this type of implant, the two membranes comprising the tested non-woven fabrics have proven to be largely biocompatible, capable of promoting healing of the injured biological tissue relatively quickly, without inducing inflammatory or rejection reactions. The membranes comprising the tested non-woven fabrics have therefore proved to be a valid support in the healing process of biological tissue injuries, capable of preventing the formation of dehiscences and incisional hernias.

## Claims

1. Membrane for re-establishing the continuity of injured biological tissues, which comprises a non-woven fabric of fibres of a polyhydroxyalkanoate (PHA) containing 3-hydroxybutyrate monomer units, wherein said fibers have an average diameter comprised from 800 nm to 3500 nm;
wherein the PHA is a poly-3-hydroxybutyrate homopolymer (P3HB);
wherein the PHA fibers of the non-woven fabric are produced by electrospinning;
wherein the non-woven fabric has dimensions comprised from 1 cm² to 50 cm², a rectangular, rhomboidal, square, oval, round or irregular shape and a thickness comprised from 10 um to 700 um.

2. Membrane according to claim 1, wherein the PHA has a weight average molecular weight (M_{w}) comprised in the range from 5,000 to 1,500,000 Da, preferably from 100,000 to 1,000,000 Da.

3. Membrane according to any one of the preceding claims, wherein at least one active ingredient is associated with the fibers of the non-woven fabric.

4. Membrane according to claim 3, wherein the at least one active ingredient is selected from: pentoxifylline, doxycycline, ibuprofen, cefazolin, rifampicin, paclitaxel, itraconazole, mefoxin, tetracycline hydrochloride or mixtures thereof.

5. Membrane according to claim 4, wherein the active ingredient is doxycycline.

6. Membrane according to claim 5, wherein the doxycycline has a concentration comprised from 0.1% w/w to 4% w/w, more preferably comprised from 0.2% w/w to 2% w/w, with respect to the weight of the PHA.

7. Method for producing a membrane comprising a non-woven fabric according to any of the previous claims, which comprises:
- preparing a spinning solution by solubilization of the polyhydroxyalkanoate (PHA) containing 3-hydroxybutyrate monomeric units in an organic solvent, wherein the PHA is a poly-3-hydroxybutyrate homopolymer (P3HB);
- subjecting the spinning solution to an electrospinning process by means of a spinneret and a rotating support placed substantially perpendicular to the spinning direction, using a configuration called "horizontal arrangement", so as to obtain the non-woven fabric.

8. Method according to claim 7, wherein the organic solvent usable for the preparation of the spinning solution is selected from 1,1,1,3,3,3-hexafluoro-2-propanol (HFIP), chloroform, N,N-dimethylformamide (DMF), or mixtures thereof.

9. Method according to claim 7 or 8, wherein in the spinning solution, the PHA containing 3-hydroxybutyrate monomer units has a concentration comprised from 1% to 20% w/v, more preferably comprised 2 to 10% w/v.

10. Method according to any one of claims 7 to 9, wherein the spinning solution further comprises at least one active ingredient.

## Patentansprüche

1. Membran zur Wiederherstellung der Kontinuität von verletzten biologischen Geweben, die einen Vliesstoff aus Fasern eines Polyhydroxyalkanoats (PHA) umfasst, das 3-Hydroxybutyrat-Monomereinheiten enthält, wobei die Fasern einen durchschnittlichen Durchmesser von 800 nm bis 3500 nm aufweisen;
wobei das PHA ein Poly-3-Hydroxybutyrat-Homopolymer (P3HB) ist;
wobei die PHA-Fasern des Vliesstoffes durch Elektrospinnen hergestellt werden;
wobei der Vliesstoff Abmessungen von 1 cm² bis 50 cm², eine rechteckige, rautenförmige, quadratische, ovale, runde oder unregelmäßige Form und eine Dicke von 10 µm bis 700 µm aufweist.

2. Membran nach Anspruch 1, wobei das PHA ein gewichtsmittleres Molekulargewicht (Mw) im Bereich von 5.000 bis 1.500.000 Da, vorzugsweise von 100.000 bis 1.000.000 Da aufweist.

3. Membran nach einem der vorstehenden Ansprüche, wobei mindestens ein Wirkstoff mit den Fasern des Vliesstoffes verbunden ist.

4. Membran nach Anspruch 3, wobei der mindestens eine Wirkstoff ausgewählt ist aus: Pentoxifyllin, Doxycyclin, Ibuprofen, Cefazolin, Rifampicin, Paclitaxel, Itraconazol, Mefoxin, Tetracyclinhydrochlorid oder Mischungen davon.

5. Membran nach Anspruch 4, wobei der Wirkstoff Doxycyclin ist.

6. Membran nach Anspruch 5, wobei das Doxycyclin eine Konzentration von 0,1 Gew.-% bis 4 Gew.-%, bevorzugter von 0,2 Gew.-% bis 2 Gew.-%, bezogen auf das Gewicht des PHA, aufweist.

7. Verfahren zur Herstellung einer Membran, die einen Vliesstoff nach einem der vorstehenden Ansprüche umfasst, das Folgendes umfasst:
- Herstellen einer Spinnlösung durch Solubilisierung des Polyhydroxyalkanoats (PHA), das 3-Hydroxybutyrat-Monomereinheiten enthält, in einem organischen Lösungsmittel, wobei das PHA ein Poly-3-Hydroxybutyrat-Homopolymer (P3HB) ist;
- Unterziehen der Spinnlösung einem Elektrospinnverfahren mit Hilfe einer Spinndüse und eines rotierenden Trägers, der im Wesentlichen senkrecht zur Spinnrichtung angeordnet ist, unter Verwendung einer als "horizontale Anordnung" bezeichneten Konfiguration, um den Vliesstoff zu erhalten.

8. Verfahren nach Anspruch 7, wobei das zur Herstellung der Spinnlösung verwendbare organische Lösungsmittel ausgewählt ist aus 1,1,1,3,3,3-Hexafluor-2-propanol (HFIP), Chloroform, N,N-Dimethylformamid (DMF) oder Mischungen davon.

9. Verfahren nach Anspruch 7 oder 8, wobei in der Spinnlösung das PHA, das 3-Hydroxybutyrat-Monomereinheiten enthält, eine Konzentration von 1% bis 20% Gewicht/Volumen, vorzugsweise von 2 bis 10% Gewicht/Volumen aufweist.

10. Verfahren nach einem der Ansprüche 7 bis 9, wobei die Spinnlösung ferner mindestens einen Wirkstoff enthält.

## Revendications

1. Membrane destinée à rétablir la continuité des tissus biologiques lésés, qui comprend une étoffe non tissée de fibres d'un polyhydroxyalcanoate (PHA) contenant des unités monomères de 3-hydroxybutyrate, dans lequel lesdites fibres ont un diamètre moyen compris entre 800 nm et 3500 nm ;
dans lequel le PHA est un homopolymère de poly-3-hydroxybutyrate (P3HB) ;
dans lequel les fibres PHA de l'étoffe non tissée sont produites par électrofilage ;
dans lequel l'étoffe non tissée a des dimensions comprises entre 1 cm² et 50 cm², une forme rectangulaire, rhomboïdale, carrée, ovale, ronde ou irrégulière et une épaisseur comprise entre 10 µm et 700 µm.

2. Membrane selon la revendication 1, dans laquelle le PHA a une masse moléculaire moyenne en poids (Mp) comprise entre 5.000 et 1.500.000 Da, de préférence entre 100.000 et 1.000.000 Da.

3. Membrane selon l'une quelconque des revendications précédentes, dans laquelle au moins un principe actif est associé aux fibres de l'étoffe non tissée.

4. Membrane selon la revendication 3, dans laquelle l'au moins un ingrédient actif au moins est choisi parmi: la pentoxifylline, la doxycycline, l'ibuprofène, la céfazoline, la rifampicine, le paclitaxel, l'itraconazole, la méfoxine, le chlorhydrate de tétracycline ou leurs mélanges.

5. Membrane selon la revendication 4, dans laquelle l'ingrédient actif est la doxycycline.

6. Membrane selon la revendication 5, dans laquelle la doxycycline a une concentration comprise entre 0,1% p/p et 4% p/p, plus préférablement comprise entre 0,2% p/p et 2% p/p, par rapport au poids du PHA.

7. Procédé de fabrication d'une membrane comprenant une étoffe non tissée selon l'une quelconque des revendications précédentes, qui comprend:
- la préparation d'une solution de filage par solubilisation du polyhydroxyalcanoate (PHA) contenant des unités monomériques de 3-hydroxybutyrate dans un solvant organique, le PHA étant un homopolymère de poly-3-hydroxybutyrate (P3HB);
- le fait de soumettre la solution de filage à un processus d'électrofilage au moyen d'une filière et d'un support rotatif placés substantiellement perpendiculairement à la direction de filage, selon une configuration appelée "disposition horizontale", afin d'obtenir l'étoffe non tissée.

8. Procédé selon la revendication 7, dans lequel le solvant organique utilisé pour la préparation de la solution de filage est choisi parmi le 1,1,1,3,3,3-hexafluoro-2-propanol (HFIP), le chloroforme, le N,N-diméthylformamide (DMF), ou leurs mélanges.

9. Procédé selon la revendication 7 ou 8, dans lequel, dans la solution de filage, le PHA contenant des unités monomère 3-hydroxybutyrate possède une concentration comprise de 1% à 20% p/v, plus de préférence comprise de 2 à 10% p/v.

10. Procédé selon l'une des revendications 7 à 9, dans lequel la solution de filage comprend en outre au moins un ingrédient actif.
